# EUROPÄISCHE PATENTSCHRIFT

(11) **EP 1 001 757 B1**
(45) Veröffentlichungstag und Bekanntmachung des Hinweises auf die Patenterteilung: **25.01.2006**
(21) Anmeldenummer: 98937580.3
(22) Anmeldetag: 21.07.1998
(51) Int. Cl.: A61K 31/19, A61K 9/20

(54) **SCHNELL WIRKSAMES ANALGETICUM**
FAST-ACTING ANALGESIC
ANTALGIQUE D'ACTION RAPIDE

(30) Priorität: 01.08.1997 DE 19733505
(43) Veröffentlichungstag der Anmeldung: 24.05.2000
(73) Patentinhaber: Abbott GmbH & Co. KG, 65205 Wiesbaden (DE)
(72) Erfinder: ZEIDLER, Jürgen, D-67112 Mutterstadt (DE); NEUMANN, Jörg, D-67117 Limburgerhof (DE); LIEPOLD, Bernd, D-68161 Mannheim (DE); ROSENBERG, Joerg, D-67158 Ellerstadt (DE); BERNDL, Gunther, D-67273 Herxheim (DE); BREITENBACH, Jörg, D-68199 Mannheim (DE); VOLLGRAF, Christiane, D-67240 Bobenheim-Roxheim (DE)
(74) Vertreter: Riedl, Peter
(86) Internationale Anmeldenummer: PCT/EP1998/004552
(87) Internationale Veröffentlichungsnummer: WO 1999/006038

(56) Entgegenhaltungen:
- WO-A-96/14058
- WO-A-97/26866
- DE-A- 19 635 676
- DATABASE CHEMABS CHEMICAL ABSTRACTS SERVICE, COLUMBUS, OHIO, US BREITENBACH, J. ET AL: "Solid dispersions by an integrated melt extrusion system" XP002085752 & PROC. INT. SYMP. CONTROLLED RELEASE BIOACT. MATER. (1998), 25TH, 804-805 CODEN: PCRMEY;ISSN: 1022-0178, 21. - 26. Juni 1998,

## Beschreibung

Die vorliegende Erfindung betrifft eine schnell wirksame analgetische Zubereitung, enthaltend als analgetisch wirksame Substanz Ibuprofen in einer Hilfsstoffmatrix, wobei die Zubereitung eine poröse Struktur und eine Dichte von grösser 1 bis 2,5 g/cm³ aufweist.

Weiterhin betrifft die Erfindung ein Verfahren zur Herstellung der Zubereitung.

Die Verwendung von Ibuprofen, der 2-(4.Isobutylphenyl)-propionsäure, als nichtsteroidales Analgeticum ist seit längerem bekannt. Ibuprofen weist ein asymmetrisches Kohlenstoffatom auf und liegt in der therapeutisch angewendeten Form im allgemeinen als Racemat vor.

Problematisch ist im Hinblick auf den in der Schmerztherapie erwünschten schnellen Wirkungseintritt die Schwerlöslichkeit des Wirkstoffs.

Aus der DE-C 36 39 038 ist bekannt, einen schnelleren Wirkungseintritt durch Einsatz des reinen S-(+)-Isomers zu erzielen.

In der DE-C 41 40 185 wird vorgeschlagen, das Problem der Schwerlöslichkeit des Ibuprofens durch die Verwendung von kolloid-dispersen Systemen auf Basis von Gelatine zu lösen.

Weiterhin wird das Problem der Schwerlöslichkeit häufig dadurch gelöst, dass das Ibuprofen in seine wasserlöslichen Salze überführt wird. Jedoch ist beispielsweise Natriumibuprofenat hygroskopisch und nur schlecht tablettierbar.

Ibuprofen wird auch als Lysinsalz angeboten, welches im Vergleich zur freien Säure einen deutlich schnelleren und höheren Blutspiegelmaximalwert Cₘₐₓ erzielt und bisher als die schnellste Darreichungsform gilt. Allerdings ist die Überführung der Säure in das Salz aufwendiger und kostenungünstiger. Hinzu kommt; dass Lysin als Aminosäure ein allergenes Potential hat, weshalb das Lysinsalz in einigen Ländern nicht als Arzneimittel zugelassen wurde.

In der WO 96/29061 ist die Herstellung transparenter fester Lösungen von Ibuprofen-Salzen durch ein Schmelzextrusionsverfahren beschrieben. Die WO 97/26866 offenbart Ibuprofen-Zubereitungen, die durch Extrusion einer Schmelze erhalten sind, die neben dem Wirkstoff N-Vinylpyrrolidon(co)polymere und pysiologisch verträgliche Natrium- oder kaliumsatze enthält. Aufgabe der vorliegenden Erfindung war es, eine schnell wirksame Zubereitung des Ibuprofens zu finden, die eine ebenso gute Wirkung wie das Lysinsalz erzielt.

Demgemäss wurden das Verfahren nach dem Auspruch 1 und die danach erhältliche Zubereitung gefunden.

Erfindungsgemäss wird Ibuprofen als freie Säure, vorzugsweise in Form des Racemats, verarbeitet. Man kann aber auch S(+)-Ibuprofen verwenden. Je nach Dosierung können die Zubereitungen 5 bis 80, vorzugsweise20 bis 60 Gew.-% an Ibuprofen enthalten. Geeignete Dosierungen sind beispielsweise 200 mg oder 400mg pro Arzneiform. Der Wirkstoff liegt vorzugsweise als als sogenannte feste Lösung in einer Hilfsstoffmatrix vor. Der Begriff "feste Lösung" ist dem Fachmann bekannt (Vgl. Chiou und Riegelmann, J. Pharm. Sci. 60(9), 1281 - 1301 (1971)).

Die Hilfsstoffmatrix enthält neben wasserlöslichen polymeren Bindemitteln Carbonate sowie gegebenenfalls übliche pharmazeutische Hilfsstoffe. Wasserlöslich bedeutet, dass sich in 100 g Wasser bei 20°C mindestens 0,5 g, vorzugsweise mindestens 2 g des Polymeren lösen, gegebenenfalls auch kolloidal.

Als polymere Bindemittel eignen sich erfindungsgemass wasserlösliche Cellulosederivate wie Hydroxyalkylcellulosen, beispielsweise Hydroxypropylcellulose, sowie insbesondere wasserlösliche Homo- und Copolymere des N-Vinylpyrrolidons (NVP)mit K-Werten im Bereich von 10 bis 90, bevorzugt K25 bis K30. Geeignete Copolymere sind beispielsweise Copolymere aus NVP und Vinylacetat, beispielsweise ein Copolymer aus 60 Gew.-% NVP und 40 Gew.-% Vinylacetat mit einem K-Wert von 28 oder 30. Besonders bevorzugt als polymeres Bindemittel ist Polyvinylpyrrolidon (PVP) mit einem K-Wert von 30 ( zur Bestimmung des K-Wertes vgl. H. Fikentscher, Cellulosechemie 13 (1932) 58-64 und 71-74). Es können auch Mischungen von Bindemitteln eingesetzt werden. Die polymeren Bindemittel können in Mengen von 10 bis 80, bevorzugt 30 bis 70 Gew.-%, bezogen auf das Gesamtgewicht der Zubereitung eingesetzt werden.

Geeignete Carbonate sind erfindungsgemäss die Alkalicarbonate Natriumcarbonat und Kaliumcarbonat sowie die Erdalkalicarbonate Calciumcarbonat und Magnesiumcarbonat. Weiterhin eignen sich auch die entsprechenden Hydrogencarbonate des Natriums und des Kaliums.

Die Carbonate oder Hydrogencarbonate können in Mengen von 0,1 bis 20, bevorzugt 2 bis 15 Gew.-%, bezogen auf die Gesamtzubereitung, eingesetzt werden. Bevorzugt werden wasserfreie Carbonate oder Hydrogencarbonate eingesetzt. Bevorzugt ist insbesondere auch der Einsatz von gemahlenen Carbonaten, wobei die Korngrössen bevorzugt kleiner 500 µm sind.

Zusätzlich können die Zubereitungen noch übliche Pharmahilfsstoffe in den hierfür üblichen Mengen enthalten, beispielsweise Stabilisatoren, Antioxidantien, Farbstoffe, Aromastoffe, Füllstoffe oder Stabilisatoren wie beispielsweise hochdisperses Siliciumdioxid oder Gleitmittel. Weiterhin können die Arzneiformen auch Codein, Coffein oder Vitamin C in den hierfür üblichen Mengen enthalten.

Die Herstellung der erfindungsgemässen Zubereitungen erfolgt durch Vermischen der Komponenten unter Einsatz von Scherkräften und Zufuhr thermischer Energie. Bevorzugt erfolgt das Mischen in einem Ein- oder Mehrschneckenextruder, besonders bevorzugt einem Zweischneckenextruder. Durch Zufuhr thermischer Energie wird eine Schmelze der Mischungskomponenten erzeugt. Dies geschieht üblicherweise durch Aufheizen des Extrudermantels auf Temperaturen im Bereich von 50 bis 180, vorzugsweise 80 bis 130°C. Das Vermischen des Wirkstoffs mit den anderen Komponenten kann vor oder nach dem Schmelzen des polymeren Bindemittels erfolgen. Die Schmelzen sind lösungsmittelfrei. Damit ist gemeint, dass kein Wasser oder organische Lösungsmittel zugesetzt wird.

Die geschmolzene Mischung der Komponenten wird durch die Schnekkenbewegung in Richtung auf den Extruderauslass, der vorzugsweise aus einer Düse besteht, gefördert. Erfindungsgemäss wird im letzten Segment oder Schuss vor der Düse ein Vakuum von 10 bis 600 mbar, bevorzugt 30 bis 200 mbar, besonders bevorzugt 50 bis 150 mbar, angelegt. Nach Extrusion durch die Düse wird die noch plastische Masse zu geeigneten Arzneiformen verformt.

Geeignete Arzneiformen sind bevorzugt Tabletten, insbesondere Bolustabletten, linsenförmige Tabletten oder aber auch Buccaltabletten, Lutschtabletten, Trinkgranulate, Granulate oder Pellets für Sachets oder zum Befüllen von Kapseln. Auch Suppositorien sind erfindungsgemäß geeignet.

Die Herstellung von Tabletten erfolgt vorzugsweise gemäss dem in der EP-A 240 906 beschriebenen Verfahren durch Hindurchführen des noch plastischen Stranges zwischen zwei gegenläufig angetriebene Walzen mit einander gegenüberliegenden Vertiefungen im Walzenmantel. Durch entsprechende Wahl der Form dieser Vertiefungen können auch Tabletten mit Bruchkerben erhalten werden. Granulate oder Pellets können durch Kaltabschlag oder, bevorzugt, durch Heissabschlag gewonnen werden.

Die Arzneiformen können zusätzlich noch mit an sich bekannten Überzügen, die keinen Einfluss auf das Freisetzungsverhalten haben, versehen werden.

Die erfindungsgemässen Arzneiformen eignen sich zur vorzugsweise oralen Verabreichung. Sie weisen eine Dichte, bestimmt mit einem Heliumpyknometer, von grösser 1 bis 2,5, bevorzugt 1,1 bis 2,0, besonders bevorzugt 1,4 bis 1,9 g/cm³ auf und sind porös. Die Dichte wird mit einem Heliumpyknometer nach OECD Guideline, Paris 1981, Test Guideline, S. 100, oder nach DIN 55990 oder DIN 53243 bestimmt. Dabei wird die Volumenverdrängung von flüssigem Helim bestimmt. Dieses Verfahren liefert im Gegensatz zu herkömmlichen Methoden die wahre Dichte eines Feststoffes und nicht die Schüttgutdichte. Das Helium kann aufgrund seines geringen atomaren Durchmessers auch in kleinste Risse und Poren eindringen.

Die mittlere Porengrösse liegt bevorzugt bei 80 µm, die Poren können Durchmesse im Bereich von 10 bis 300 µm aufweisen. Im Querschnitt durch eine Arzneiform ist eine wabenartige Struktur zu erkennen.

Der Wirkstoff liegt besonders bevorzugt als feste Lösung in der Matrix vor, was mit Hilfe von DSC-Messungen (Differential Scanning Calorimetry) und durch Röntgenbeugungsuntersuchungen nachgewiesen werden kann. Die Arzneiformen können aber auch als Mischformen vorliegen, in denen ein Teil des Wirkstoffs als feste Lösung vorliegt und ein anderer Teil rekristallisiert ist. Der Wirkstoff kann auch vollständig rekristallisiert vorliegen. Man kann durch die Menge an zugefügtem Carbonat den Anteil an rekristallisierter freier Säure steuern.

Im Gegensatz zu bekannten festen Lösungen des Ibuprofens sind die erfindungsgemässen Arzneiformen jedoch nicht transparent, sondern im Aussehen opak.

Die Freisetzungsrate für den Wirkstoff nach dem USP-Drehkörbchen-Modell nach USP 23 beträgt mindestens 95 % nach 10 min.

Die erfindungsgemässen Zubereitungen sind nicht nur schnell freisetzend, sondern auch schnell wirksam. Die Zeit (tₘₐₓ) bis zum Erreichen des maximalen Blutplasmaspiegels (Cₘₐₓ) liegt im Bereich von 0,5 Stunden.

Die AUC(area under the curve)-Werte der erfindungsgemässen Arzneiformen, also die Fläche unter der Konzentrations-Zeit-Kurve, die ein Maß für die Substanzmenge im Organismus darstellen, sind weitgehend vergleichbar mit denen eines handelsüblichen schnellwirksamen Ibuprofen-Lysinats.

Es war im Hinblick auf den Stand der Technik völlig überraschend, dass mit den erfindungsgemässe Arzneiformen eine zu Lysinaten bioäquivalente Form gefunden werden konnte.

Überraschend war auch, dass bei Anlegen eines Vakuums vor dem Extruderauslass poröse Formen mit einer Dichte grösser 1 g/cm³ erhalten wurden.

### Beispiele

Die in den nachstehenden Beispielen jeweils angegeben Zusammensetzungen wurden vorgemischt und in den Einzug eines Doppelschneckenextruders (ZSK-40, Werner & Pfleiderer) eingetragen. Die Extrusion erfolgte mit einem Produktdurchsatz von 25 kg pro Stunde bei einer Schneckendrehzahl von 90 Upm. Die Temperaturen der einzelnen Zonen ("Schüsse") des Extruders sowie der beheizten Düsenleiste betrugen:
Schuss 1: 80°C, Schuss 2: 120°C, Schuss 3: 130°C, Schuss 4: 130°C, Kopf: 130°C, Düse 130°C. In Schuss 4 wurde ein Vakuum von 51,5 mbar angelegt. Aus dem Extrudat wurde gemäss dem in der EP-A 240 906 beschriebenen Kalandrierverfahren Bolus-Tabletten hergestellt.

Die Wirkstofffreisetzung wurde mittels der Drehkörbchen-Methode (Basket-Methode nach USP XXIII, US-Arzneibuch) gemessen. Die Bestimmung erfolgt bei 37°C im no-change-Test bei einem pH-Wert von 7,2 bei 150 Upm. Als Prüfmedium wurde eine 0,05 molare wässrige Kaliumdihydrogenphosphatlösung eingesetzt, die mit Natronlauge auf pH 7,2 eingestellt wurde. Eine entsprechende Menge Arzneiform wurde eingewogen. Die Gehaltsbestimmung erfolgte photometrisch mittels Ableitungsspektrum bei 256 bis 270 nm mit externer Standardkalibrierung.

Die Tabletten wurden mit einem kommerziell erhältlichen Überzug(Opadry® OY-S-24939 der Firma Colorcon), einer 15 gew.-%igen wässrigen Dispersion der folgenden Zusammensetzung versehen: 58,04 % Hydroxypropylmethylcellulose (HPMC)mit einer Viskosität von 6 mPas, 5,76 % HPMC mit einer Viskosität von 15 mPas, 5,76 % Hydroxypropylcellulose, 11,16 % Talkum, 9 % Polyethylenglykol (PEG) 400, 1,61 % PEG 6000, 8,18 % Titandioxid, 0,19 % Eisenoxid rot, 0,15 % hochdisperses Siliciumdioxid, 0,15 % Natriumdocusat (% jeweils Gew.-%). Die Beschichtung erfolgte auf bekannte Weise durch Besprühen im Dragierkessel.

Die Bestimmung der Dichte erfolgte in flüssigem Helium mit einem Ultrapycnometer 1000 der Firma Quantachrome Corp..

### Beispiel 1

| | |
|---|---|
| PVP K 30 | 55,07 Gew.-% |
| Copolyvidon* K28 | 10,89 Gew.-% |
| Na₂CO₃ wasserfrei nach DAB | 10,00 Gew.-% |
| Ibuprofen | 23,53 Gew.-% |
| hochdisperses Siliciumdioxid | 0,51 Gew.-% |

| | |
|---|---|
| (* Copolymer aus 60Gew.-% Vinylpyrrolidon und 40 Gew.-% Vinylacetat) | |

Tablettengewicht: 850 mg ohne Überzug, Überzug 15 mg, Ibuprofen-Dosis 200 mg,
Freisetzung nach 10 min 100 %
Dichte des unbeschichteten Tablettenkerns 1,573 g/cm³

### Beispiel 2

| | |
|---|---|
| PVP K 30 | 55,50 Gew.-% |
| Na₂CO₃, wasserfrei | 12,00 Gew.-% |
| Ibuprofen | 32,00 Gew.-% |
| hochdisperses Siliciumdioxid | 0,5 Gew.-% |

Tablettengewicht: 650 mg, Überzug 15 mg,
Ibuprofen-Dosis 200 mg
Freisetzung nach 10 min 100 %
Dichte des unbeschichteten Tablettenkerns 1,841 g/cm³

### Beispiel 3

| | |
|---|---|
| PVP K30 | 41,00 Gew.-% |
| Na₂CO₃ wasserfrei | 12,00 Gew.-% |
| Ibuprofen | 47,00 Gew.-% |

Tablettengewicht: 850 mg ohne Überzug
Ibuprofen-Dosis 400 mg

### Bestimmung der pharmakokinetischen Parameter

Die Studie wurde mit Einzeldosis (200 mg) bei dreifachem Crossover bei gesunden Männern durchgeführt.

Verabreicht wurde eine Arzneiform gemäss Beispiel 1. Zum Vergleich wurde ein kommerziell erhältliches Ibuprofen-Lysinat (Dolormin® Filmtabletten, 342 mg Ibuprofen-D,L-Lysinsalz, entspricht 200 mg Ibuprofen) verabreicht.

**Tabelle**

| Pharmakokinetische Parameter | Beispiel 1 | Dolormin |
|---|---|---|
| AUC [mg*h/L] | 62,78 | 57,41 |
| Cₘₐₓ [mg/L] | 22,77 | 23,19 |
| AUC₀₋₁ₕ [mg*h/L] | 15,23 | 15,55 |
| tₘₐₓ [h] | 0,50 | 0,50 |

Angegeben ist jeweils das geometrische Mittel (n=12).

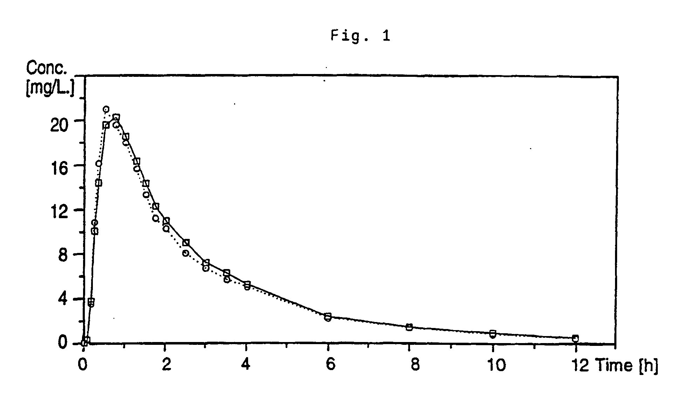

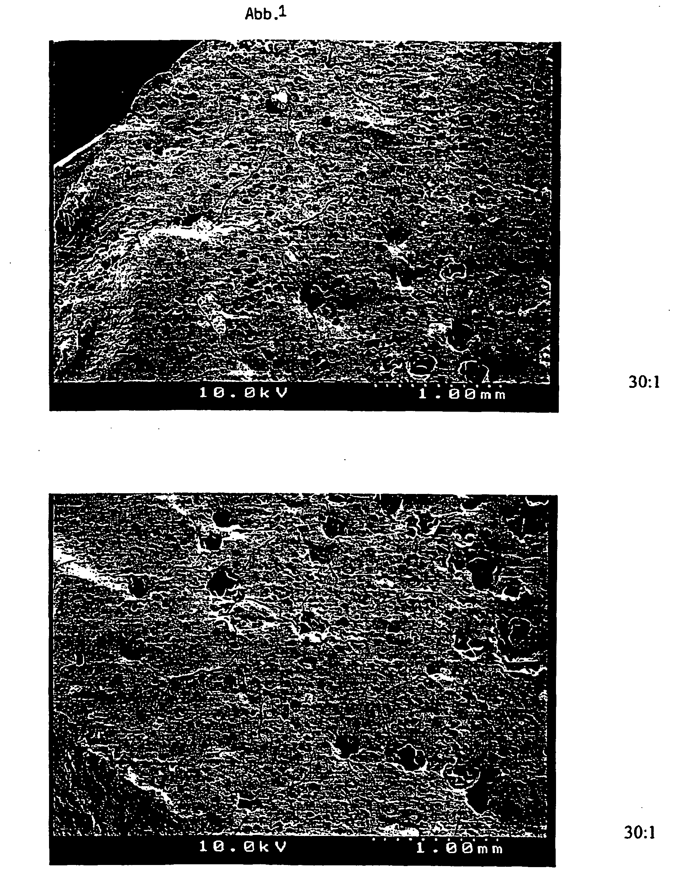

In der Figur ist der Verlauf der Blutplasmakurve über einen Zeitraum von 12 Stunden abgebildet. Dabei ist die Plasmakonzentration in [mg/L] gegen die Zeit in [h] als geometrisches Mittel, n=12, aufgetragen.
-□- Ibuprofen 200 mg Filmtablette gemäss Beispiel 1
...○... Dolormin Filmtablette

Der Kurvenverlauf ist weitestgehend identisch.

Weiterhin wurde eine elektronenmikroskopische Untersuchung der Bruchfläche einer unbeschichteten Tablette gemäss Beispiel 1 durch geführt. Die Untersuchung erfolgte an einer Metallbedampften Bruchfläche.

In der Abbildung sind deutlich die zu der Bruchfläche hin offenen Poren zu erkennen. Ebenfalls sind die nicht offenen Poren als kreisrunde kleine Vertiefungen zu erkennen.

## Patentansprüche

1. Verfahren zur Herstellung eines Analgeticums, das als analgetisch wirksame Substanz Ibuprofen in einer Hilfsstoffmatrix enthält, mit einer porösen Struktur und einer Dichte von größer 1 bis 2,5 g/cm³ durch Vermischen der analgetisch wirksamen Substanz mit Matrixhilfsstoffen, wobei die Matrixhilfsstoffe ein thermoplastisch verarbeitbares, wasserlösliches polymeres Bindemittel und ein Carbonat, das ausgewählt ist unter Alkali- oder Erdalkalicarbonaten oder Hydrogencarbonaten des Natriums und Kaliums, enthalten, unter Anwendung von Scherkräften und Extrusion durch eine Düse mit anschließender Formgebung, **dadurch gekennzeichnet, dass** man die plastifizierte Mischung vor der Extrusion durch die Düse einem Vakuum aussetzt.

2. Verfahren nach Anspruch 1, **dadurch gekennzeichnet, dass** das polymere Bindemittel ein Homo- oder Copolymer des N-Vinylpyrrolidons ist.

3. Verfahren nach Anspruch 1 oder 2, **dadurch gekennzeichnet, dass** die analgetisch wirksame Substanz Ibuprofen als Racemat ist.

4. Schnell wirksames Analgeticum, erhältlich nach dem Verfahren nach einem der Ansprüche 1 bis 3.

5. Analgeticum nach Anspruch 4, mit einer Dichte von 1,4 bis 1,9 g/cm³.

## Claims

1. Method of preparing an analgesic which contains ibuprofen as the analgesically active substance in an excipient matrix, having a porous structure and a density of more than 1 to 2.5 g/cm³, by mixing the analgesically active substance with matrix excipients, the matrix excipients containing a thermoplastically workable, water-soluble polymeric binder and a carbonate selected from among the alkali or alkaline earth carbonates or hydrogen carbonates of sodium and potassium, with the use of shear forces and extrusion through a die followed by shaping, **characterised in that** the plasticised mixture is exposed to a vacuum before being extruded through the die.

2. Method according to claim 1, **characterised in that** the polymeric binder is a homo- or copolymer of N-vinylpyrrolidone.

3. Method according to claim 1 or 2, **characterised in that** the analgesically active substance ibuprofen is in the form of the racemate.

4. Fast-acting analgesic, obtainable by the method according to one of claims 1 to 3.

5. Analgesic according to claim 4, having a density of 1.4 to 1.9 g/cm³.

## Revendications

1. Procédé de fabrication d'un analgésique qui contient, dans une matrice d'excipient, de l'ibuprofène en tant que substance à action analgésique, ayant une structure poreuse et une masse volumique supérieure à 1 à 2,5 g/cm³, par mélange de la substance à action analgésique avec des excipients matriciels, les excipients matriciels contenant un liant polymère hydrosoluble pouvant être travaillé par voie thermoplastique, et un carbonate choisi parmi les carbonates alcalins ou alcalino-terreux ou des hydrogénocarbonates de sodium et de potassium, en appliquant des forces de cisaillement et par extrusion à travers une tuyère avec façonnage consécutif, **caractérisé en ce qu'**on expose le mélange plastifié à un vide avant l'extrusion par la tuyère.

2. Procédé selon la revendication 1, **caractérisé en ce que** le liant polymère est un homopolymère ou un copolymère de la N-vinylpyrrolidone.

3. Procédé selon la revendication 1 ou 2, **caractérisé en ce que** la substance à action analgésique est de l'ibuprofène en tant que racémate.

4. Analgésique à action rapide, pouvant être obtenu avec le procédé selon l'une quelconque des revendications 1 à 3.

5. Analgésique selon la revendication 4, ayant une masse volumique de 1,4 à 1,9 g/cm³.
